# EUROPEAN PATENT SPECIFICATION

(11) **EP 2 366 379 B1**
(45) Date of publication and mention of the grant of the patent: **08.04.2015**
(21) Application number: 11156115.5
(22) Date of filing: 25.02.2011
(51) Int. Cl.: A61K 9/16, A61K 31/546

(54) **Cefdinir formulation with improved dissolution rate**
Cefdinir-Formulierung mit verbesserter Auflösungsgeschwindigkeit
Forme de cefdinir avec taux de dissolution amélioré

(30) Priority: 25.02.2010 TR 201001417
(43) Date of publication of application: 21.09.2011
(73) Proprietor: Sanovel Ilaç Sanayi Ve Ticaret Anonim Sirketi, 34460 Sariyer/Istanbul (TR)
(72) Inventor: Toksöz, Ahmet, 34398, Istanbul (TR); Cifter, Ümit, 34398, Istanbul (TR); Türkyilmaz, Ali, 34398, Istanbul (TR); Üzer, Ibrahim Murat, 34398, Istanbul (TR); Palantöken, Arzu, 34398, Istanbul (TR)
(74) Representative: Sevinç, Erkan

(56) References cited:
- EP-A1- 0 890 359
- EP-A1- 1 795 197
- EP-A1- 1 905 432
- WO-A1-02/067943
- US-A1- 2006 122 165

## Description

### Field of Invention

The present invention relates to formulations of cefdinir or a pharmaceutically acceptable salt of cefdinir. The present invention more particularly relates to formulations, enabling to increase the dissolution rate and bioavailability of cefdinir.

### Background of Invention

Cefdinir, with the chemical name (6R,7R)-7-[(Z)-2-(2-aminothiazol-4-yl)-2-hydroxy-iminoglioxylamidoi]-8-oxo-3-vinyl-5-thia-1-azabicyclo[4.2.0]oct-2-ene-2-carbonic acid, is a broad-spectrum semisynthetic cephalosporin. Its chemical structure is illustrated with Formula I given below.

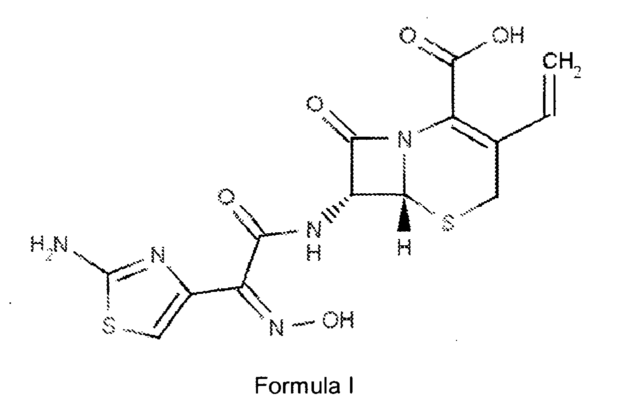

Cefdinir was originally disclosed with the application US 4,559,334.

The patent application EP 1 905 432 discloses a stable nanoparticulate composition, comprising cephalosporin of a diameter size less than 2000 nm and at least one surface stabilizer adsorbed on the surface of particles, said surface stabilizer being free of intermolecular cross-linkages.

The patent application WO2005060936 protects an oral suspension formulation containing cefdinir.

The patent EP 0 890 359 B1 protects a tablet formulation containing beta-lactam antibiotics, including, *inter alia,* cefdinir. According to this formulation, a tablet contains 60 to 85% by weight of an beta-lactam antibiotic, 1 to 10% by weight of hydroxypropyl cellulose and/or cross-linked polyvinylpyrrolidone, hydroxypropyl cellulose, or hydroxypropyl methyl cellulose.

The patent US2006233878 discloses a controlled-release formulation comprising beta-lactam antibiotic and one or more carbomers.

US2006122165 A1 discloses a pharmaceutical compositions comprising a crystalline form of cefdinir and processes for preparing them.

Cefdinir is a solid, with a white or whitish yellow color. Practically, it is water-insoluble. Its lower solubility and dissolution rate reduce the bioavailability of cefdinir.

A certain period of time is required to achieve efficiency of current formulations. Considering this problem, it is obvious that a novelty is needed in the relevant art of formulations comprising cefdinir.

### Object and Brief Description of Invention

The present invention provides a cefdinir formulation, eliminating all aforesaid problems and brining additional advantages to the relevant prior art.

Accordingly, the main object of the present invention is to obtain a stable cefdinir formulation with improved bioavailability.

Another object of the present invention is to obtain a formulation by which the solubility and dissolution rate of cefdinir is enhanced.

A pharmaceutical formulation comprising cefdinir or a pharmaceutically acceptable salt or polymorph of cefdinir has been developed to carry out all objects, referred to above and to emerge from the following detailed description.

According to a preferred embodiment of the present invention, said novelty is carried out with a pharmaceutically acceptable salt or polymorph of cefdinir, which is wet-granulated and has an average particle size of 10 µm at most, and calcium carboxymethyl cellulose. The particle size of cefdinir is between 1 to 10 µm.

According to a preferred embodiment of the present invention, the proportion by weight of cefdinir to calcium carboxymethyl cellulose is the range of 3 to 17.

According to another preferred embodiment of the present invention, the proportion by weight of cefdinir to calcium carboxymethyl cellulose is the range of 4 to 15.

According to a further preferred embodiment of the present invention, the proportion by weight of cefdinir to calcium carboxymethyl cellulose is the range of 5 to 12.

A preferred embodiment according to the present invention further comprises polyoxyethylene stearate, as a surface active agent.

A preferred embodiment according to the present invention further comprises colloidal silicone dioxide, as a glidant.

A preferred embodiment according to the present invention further comprises magnesium stearate, as a lubricant.

A further preferred embodiment according to the present invention provides a method for preparing a pharmaceutical formulation, this method comprising the steps of
a. adding calcium carboxymethyl cellulose and polyoxyethylene stearate to cefdinir and mixing the resulting mixture,
b. forming wet granulation with alcohol and water,
c. drying the granules,
d. adding colloidal silicone dioxide and mixing the resulting mixture,
e. sieving dried granules,
f. mixing magnesium stearate together with the granules,
g. filling the granules into capsules
wherein the proportion by weight of cefdinir to calcium carboxymethyl cellulose is in the range of 3 to 17.

In a further preferred embodiment of the present invention, said pharmaceutical formulation contains the following ingredients only:
a. cefdinir or a pharmaceutically acceptable salt thereof at 50 to 90% by weight,
b. calcium carboxymethyl cellulose at 5 to 20% by weight,
c. polyoxyethylene stearate at 0.1 to 5% by weight,
d. silicone dioxide at 0.5 to 5% by weight,
e. magnesium stearate at 0.5 to 3% by weight.
wherein the proportion by weight of cefdinir to calcium carboxymethyl cellulose is in the range of 3 to 17.

### Detailed Description of Invention

### Example

| **Content** | **Amount (mg)** |
|---|---|
| cefdinir | 300 |
| calcium carboxymethyl cellulose | 44.25 |
| polyoxyl 40 stearate (finely-sieved) | 2 |
| colloidal silicone dioxide | 1.875 |
| magnesium stearate | 1.875 |
| total weight | 350 |

Calcium carboxymethyl cellulose and polyoxyethylene stearate are added to cefdinir the resulting mixture is mixed. Wet granulation is formed with ethyl alcohol and water, then the granules are dried. Thereafter, colloidal silicone dioxide is added into the granules and they are mixed. Then, dried granules are sieved and magnesium stearate is added into and mixed together with the granules. Finally, the granules are filled into capsules.

Desired results are obtained with this formulation developed, thanks to keeping the average particle size of cefdinir therein by 10 µm at most, obtaining the formulation by wet granulation, and maintaining the amount of calcium carboxymethyl cellulose at the proportions specified.

The dissolution rate of different formulations containing varying amounts of calcium carboxymethyl cellulose at pH 4.5 and 0.1 N HCL medium is given hereunder.

| **Time** | **Formulation containing 44.25 mg calcium carboxymethyl cellulose** | **Formulation containing 27.20 mg calcium carboxymethyl cellulose** | **Formulation containing 44.25 mg calcium carboxymethyl cellulose** | **Formulation containing 27.20 mg calcium carboxymethyl cellulose** |
|---|---|---|---|---|
| **(minute)** | **pH: 4.5** | **pH: 4.5** | **0.1 N HCL** | **0.1 N HCL** |
| **0** | 0.0 | 0.0 | 0.0 | 0.0 |
| **5** | 68.2 | 45.8 | 77.7 | 62.6 |
| **10** | 84.2 | 75.6 | 85.9 | 73.5 |
| **15** | 88.0 | 80.3 | 88.8 | 80.3 |
| **20** | 90.2 | 83.1 | 91.4 | 84.6 |
| **30** | 90.9 | 86.1 | 93.3 | 90.4 |
| **45** | 93.2 | 88.0 | 96.0 | 94.9 |

Using 44.25 to 60 mg calcium carboxymethyl cellulose in the formulation yields the best dissolution profiles. When 20 to 30 mg calcium carboxymethyl cellulose is used in formulations, lower dissolution rates are obtained, as shown in the results.

The difference in dissolution rate within the first 15 minutes of the study is extremely critical with respect to Cmax, which is one of the criteria of bioavailability.

**Table 1**

| **Lot1** | | | **Lot2** | |
|---|---|---|---|---|
| **10 MICRON CEFDINIR (WET GRANULATION)** | | | **70 MICRON CEFDINIR (SLUG COMPRESSION)** | |
| Cefdinir | 300mg | | Cefdinir | 300mg |
| Ca CMC | 44.25 mg | | Ca CMC | 44.25 mg |
| Polyoxyl 40 stearat | 2 mg | | Polyoxyl 40 stearat | 2 mg |
| Aerosil 200 | 1.875 mg | | Aerosil 200 | 1.875 mg |
| Mg stearat | 1.875 mg | | Mg stearat | 1.875 mg |
| TOTAL | 350 mg | | TOTAL | 350 mg |

**Table 2**

| **Lot 1** | | | **Lot2** | |
|---|---|---|---|---|
| **10 MICRON CEFDINIR (WET GRANULATION)** | | | **70 MICRON CEFDINIR (SLUG COMPRESSION)** | |
| Time (minute) | Dissolution rate | | Time (minute) | Dissolution rate |
| 5. | %71 | | 5. | %43.6 |
| 10. | %86 | | 10. | %63.3 |
| 15. | %89 | | 15. | %72.8 |
| 20. | %91 | | 20. | %76.4 |
| 30 | %92 | | 30 | %82.2 |
| 45 | %94 | | 45 | %85.6 |
| | | | 60 | %87.2 |

| | | | | |
|---|---|---|---|---|
| Lot 1 : 10 µm wet granulated formulation Lot 2 : 70 µm slug compression formulation Lot 1 covers 10 µm cefdinir. It is manufactured by wet granulation. Lot 2 covers 70 µm cefdinir. It is manufactured by slug compression | | | | |

The dissolution tests were performed this condition; 0.1 N HCL ; 900ml ; basket; 50 rpm ; 37 °C. As a result of this comparative study, it is surprisingly found that the dissolution results of lot 1 is better than the dissolution results of lot 2.

With the invention realized, stable cefdinir formulations can be obtained which have surprisingly good solubility and dissolution rates, and thus high bioavailability.

This formulation is used in treating bronchitis, pharyngitis, otitis media, pneumonia, sinusitis, tonsillitis, bacterial vaginosis, and tissue infection diseases.

## Claims

1. A pharmaceutically formulation in the form of a capsule, comprising a pharmaceutically acceptable salt or polymorph of cefdinir, which is wet-granulated and has an average particle size in the range of 1-10 µm, and calcium carboxymethyl cellulose, wherein the proportion by weight of cefdinir to calcium carboxymethyl cellulose is in the range of 3 to 17.

2. A pharmaceutical formulation according to claim 1, wherein the proportion by weight of cefdinir to calcium carboxymethyl cellulose is in the range of 4 to 15.

3. A pharmaceutical formulation according to any of the preceding claims, wherein the proportion by weight of cefdinir to calcium carboxymethyl cellulose is in the range of 5 to 12.

4. A pharmaceutical formulation according to any of the preceding claims, further comprising polyoxyethylene stearate, as a surface active agent.

5. A pharmaceutical formulation according to any of the preceding claims, further comprising colloidal silicone dioxide, as a glidant.

6. A pharmaceutical formulation according to any of the preceding claims, comprising magnesium stearate, as a lubricant.

7. A method for preparing a pharmaceutical formulation according to any of the preceding claims, comprising the steps of
a. adding calcium carboxymethyl cellulose and polyoxyethylene stearate to cefdinir having average particle size in the range of 1-10 µm, and mixing the resulting mixture, wherein the proportion by weight of cefdinir to calcium carboxymethyl cellulose is provided in the range of 3 to 17,
b. forming wet granulation with alcohol and water,
c. drying the granules,
d. adding colloidal silicone dioxide and mixing the resulting mixture,
e. sieving dried granules,
f. mixing magnesium stearate together with the granules,
g. filling the granules into capsules.

8. A pharmaceutical formulation according to any of the preceding claims, comprising the following ingredients only:
a. cefdinir or a pharmaceutically acceptable salt thereof at 50 to 90% by weight,
b. calcium carboxymethyl cellulose at 5 to 20% by weight,
c. polyoxyethylene stearate at 0.1 to 5% by weight,
d. silicone dioxide at 0.5 to 5% by weight,
e. magnesium stearate at 0.5 to 3% by weight.

9. The pharmaceutical formulation as claimed in any of the preceding claims for use as a medicament for treatment of bronchitis, pharyngitis, otitis media, pneumonia, sinusitis, tonsillitis, bacterial vaginosis, and tissue infection diseases.

## Patentansprüche

1. Pharmazeutische Formulierung in Form einer Kapsel, umfassend ein pharmazeutisch verträgliches Salz oder Polymorph von Cefdinir, das nassgranuliert ist und eine mittlere Teilchengröße im Bereich von 1-10 µm aufweist, und Calciumcarboxymethylcellulose, wobei das Gewichtsverhältnis von Cefdinir zu Calciumcarboxymethylcellulose im Bereich von 3 bis 17 liegt.

2. Pharmazeutische Formulierung nach Anspruch 1, wobei das Gewichtsverhältnis von Cefdinir zu Calciumcarboxymethylcellulose im Bereich von 4 bis 15 liegt.

3. Pharmazeutische Formulierung nach einem der vorangehenden Ansprüche, wobei das Gewichtsverhältnis von Cefdinir zu Calciumcarboxymethylcellulose im Bereich von 5 bis 12 liegt.

4. Pharmazeutische Formulierung nach einem der vorangehenden Ansprüche, weiterhin umfassend Polyoxyethylenstearat als grenzflächenaktives Mittel.

5. Pharmazeutische Formulierung nach einem der vorangehenden Ansprüche, weiterhin umfassend kolloidales Siliciumdioxid als Gleitmittel.

6. Pharmazeutische Formulierung nach einem der vorangehenden Ansprüche, umfassend Magnesiumstearat als Schmiermittel.

7. Verfahren zur Herstellung einer pharmazeutischen Formulierung nach einem der vorangehenden Ansprüche, umfassend die folgenden Schritte:
a) Zugeben von Calciumcarboxymethylcellulose und Polyoxyethylenstearat zu Cefdinir mit einer mittleren Teilchengröße im Bereich von 1-10 µm und Mischen des so erhaltenen Gemischs, wobei das Gewichtsverhältnis von Cefdinir zu Calciumcarboxymethylcellulose im Bereich von 3 bis 17 liegt,
b) Ausbilden einer Nassgranulierung mit Alkohol und Wasser,
c) Trocknen der Granulatkörner,
d) Zugeben von kolloidalem Siliciumdioxid und Mischen des so erhaltenen Gemischs,
e) Sieben der getrockneten Granulatkörner,
f) Zusammenmischen von Magnesiumstearat mit den Granulatkörnern,
g) Füllen der Granulatkörner in Kapseln.

8. Pharmazeutische Formulierung nach einem der vorangehenden Ansprüche, umfassend nur die folgenden Inhaltsstoffe:
a) Cefdinir oder ein pharmazeutisch verträgliches Salz davon zu 50 bis 90 Gew.-%,
b) Calciumcarboxymethylcellulose zu 5 bis 20 Gew.-%,
c) Polyoxyethylenstearat zu 0,1 bis 5 Gew.-%,
d) Siliciumdioxid zu 0,5 bis 5 Gew.-%,
e) Magnesiumstearat zu 0,5 bis 3 Gew.-%.

9. Pharmazeutische Formulierung, wie beansprucht in einem der vorangehenden Ansprüche, zur Verwendung als Medikament zur Behandlung von Bronchitis, Pharyngitis, Mittelohrentzündung, Lungenentzündung, Sinusitis, Mandelentzündung, bakterieller Vaginose und Gewebeinfektionen.

## Revendications

1. Formulation pharmaceutique en forme de capsule comprenant un sel ou un polymorphe pharmaceutiquement acceptable de cefdinir, qui est granulé par voie humide et présente une grosseur de particule moyenne comprise dans la plage de 1 à 10 µm et de la carboxyméthylcellulose de calcium, formulation, dans laquelle la proportion en poids de cefdinir par rapport à la carboxyméthylcellulose se situe dans la plage de 3 à 17.

2. Formulation pharmaceutique suivant la revendication 1, dans laquelle la proportion en poids de cefdinir par rapport à la carboxyméthylcellulose de calcium se situe dans la plage de 4 à 15.

3. Formulation pharmaceutique suivant une des revendications précédentes, dans laquelle la proportion en poids de cefdinir par rapport à la carboxyméthylcellulose de calcium se situe dans la plage de 5 à 12.

4. Formulation pharmaceutique suivant une quelconque des revendications précédentes comprenant, en outre, du stéarate de polyoxyéthylène comme agent tensioactif.

5. Formulation pharmaceutique suivant une quelconque des revendications précédentes comprenant, en outre, un dioxyde de silicium colloïdal comme agent de glissement.

6. Formulation pharmaceutique suivant une quelconque des revendications précédentes comprenant du stéarate de magnésium comme lubrifiant.

7. Procédé de préparation d'une formulation pharmaceutique suivant une quelconque des revendications précédentes comprenant les étapes suivantes qui consistent à :
a. ajouter de la carboxyméthylcellulose de calcium et du stéarate de polyoxyéthylène au cefdinir ayant une grosseur de particule comprise dans la plage de 1 à 10 µm et mélanger le mélange qui en résulte, procédé, dans lequel la proportion en poids de cefdinir par rapport à la carboxyméthylcellulose de calcium est prévue dans la plage de 3 à 17,
b. former une granulation par voie humide avec de l'alcool et de l'eau,
c. sécher les granules,
d. ajouter du dioxyde de silicium colloïdal et mélanger le mélange qui en résulte,
e. tamiser les granules séchées,
f. mélanger le stéarate de magnésium avec les granules,
g. mettre les granules en capsules.

8. Formulation pharmaceutique suivant une quelconque des revendications précédentes comprenant seulement les ingrédients suivants :
a. cefdinir ou un sel pharmaceutiquement acceptable de celui-ci à raison de 50 à 90% en poids,
b. carboxyméthylcellulose de calcium à raison de 5 à 20% en poids,
c. stéarate de polyoxyéthylène à raison de 0,1 à 5% en poids,
d. dioxyde de silicium à raison de 0,5 à 5% en poids,
e. stéarate de magnésium à raison de 0,5 à 3% en poids.

9. Formulation pharmaceutique suivant une quelconque des revendications précédentes destinée à une utilisation comme médicament pour le traitement de la bronchite, de la pharyngite, de l'otite moyenne, de la pneumonie, de la sinusite, de la tonsilite, de la vaginose bactérienne et de maladies d'infection des tissus.
